# EUROPEAN PATENT SPECIFICATION

(11) **EP 2 019 719 B1**
(45) Date of publication and mention of the grant of the patent: **07.03.2012**
(21) Application number: 07704203.4
(22) Date of filing: 29.01.2007
(51) Int. Cl.: A61N 2/02, A61N 2/00

(54) **DEVICE FOR THE REGENERATION AND PREVENTION OF DEGENERATION OF THE CARTILAGINOUS TISSUE AND SUBCHRONDRAL BONE AND THE PROLIFERATION OF CHONDROCYTES BY MEANS OF A PULSED ELECTROMAGNETIC FIELD**
VORRICHTUNG ZUR REGENERATION UND PRÄVENTION VON DEGENERATION DES KNORPELGEWEBES UND DER SUBCHONDRIALEN KNOCHEN UND DER PROLIFERATION VON CHONDROZYTEN DURCH EIN GEPULSTES ELEKTROMAGNETISCHES FELD
DISPOSITIF POUR LA RÉGÉNÉRATION ET LA PRÉVENTION DE LA DÉGÉNÉRESCENCE DU TISSU CARTILAGINEUX ET DE L'OS SOUS-CHONDRAL ET LA PROLIFÉRATION DE CHONDROCYTES À L'AIDE D'UN CHAMP ÉLECTROMAGNÉTIQUE PULSÉ

(30) Priority: 12.05.2006 IT TO20060344
(43) Date of publication of application: 04.02.2009
(73) Proprietor: IGEA S.p.A., 41012 Carpi (IT)
(72) Inventor: SETTI, Stefania, 41012 Carpi (IT); CADOSSI, Ruggero, 41012 Carpi (IT)
(74) Representative: Bongiovanni, Simone
(86) International application number: PCT/EP2007/050849
(87) International publication number: WO 2007/131809

(56) References cited:
- EP-A1- 0 995 463
- US-B1- 6 858 000
- US-B1- 7 160 241

## Description

### TECHNICAL FIELD

The present invention relates to a device for the regeneration and prevention of degeneration of the cartilaginous tissue and subchrondral bone and the proliferation of chondrocytes by means of a pulsed electromagnetic field.

### BACKGROUND ART

Techniques are known for therapeutic treatment of the human body by means of pulsed electromagnetic fields in which a solenoid is powered by a time-variable electrical signal (for example a current-variable signal) for the generation of a pulsed electromagnetic field which is addressed towards a portion of human body containing cartilaginous tissue/subchondral bone in which induced microcurrents form which contribute to the healing and/or improvement of inflammatory processes and/or lesions present in the portion of human body treated.

Scientific observations have been reported which suggest the possibility of useful application of pulsed electromagnetic fields for the treatment of articular cartilage and subchondral bone.

Said techniques have not succeeded, however, in producing devices that can be used for a real therapeutic cycle of the cartilaginous tissues /subchondral bone for application in humans, i.e. able to act and modify in a sensitive, specific and significant way the structure of the damaged or inflamed cartilage and subchondral bone.

The lack of adequate preclinical experimentation permitting identification and combination of the optimal, i.e. effective, electromagnetic field parameters (amplitude, waveform frequency and duration of exposure) can result in incorrect choice of the parameters that characterise the electromagnetic field, therefore not obtaining any therapeutic effect, as also highlighted in literature (Fini M et al, Effects of pulsed electromagnetic fields on articular hyaline cartilage: review of experimental and clinical studies.Biomed Pharmacother.2005 Aug;59(7):388-94. Review), or where often the only effect observed is related to a reduction in the pain symptomatology, without any attempt to modify the trophism of the articular cartilage. Thansborg G et al., Treatment of knee osteoarthritis with pulsed electromagnetic fields: a randomized, double-blind, placebo-controlled study. Osteoarthritis and Cartilage. 2005 Jul;13(7):575-81. Peroz I et al, A multicenter clinical trial on the use of pulsed electromagnetic fields in the treatment of temporomandibular disorders (J Prosthet Dent. 2004 Feb;91(2):180-7.).

Patent document US-B1-6 858 000 discloses the preamble of claim 1.

### DISCLOSURE OF INVENTION

The object of the present invention is to provide a device capable of generating a pulsed electromagnetic field, the parameters of which are chosen and optimised on the basis of preclinical studies which evaluate the regeneration and prevention of degeneration of the cartilaginous tissue and subchondral bone; said preclinical studies also evaluate the proliferation of chondrocytes, in order for the device to be usable in a therapeutic type process which obtains tangible results that are clinically relevant and applicable to humans.

The device can also be used in conjunction (before or after) with the administration of pharmacological agents (drugs, growth factors) aimed at stimulating cartilaginous repair, or also in conjunction with treatment of the subchondral bone by means of microfractures.

The preceding object is achieved by the present invention since it relates to a device for the regeneration and prevention of degeneration of the cartilage and subchondral bone and the proliferation of chondrocytes by means of electromagnetic waves comprising: means for generating a periodic signal u(t); power amplification means suitable for applying said signal u(t) to at least one solenoid for the generation of a pulsed electromagnetic field M(t) addressed towards a portion of human/animal tissue containing cartilage, characterised in that it comprises setting means configured to generate an electromagnetic field having peak intensity between 0.5 and 2 milliTesla.

The present disclosure also relates to a method for the treatment and/or prevention of pathologies affecting the cartilage and/or subchondral bone and for the proliferation of chondrocytes by means of electromagnetic waves comprising the following phases: generate a periodic signal u(t); apply said signal u(t) to at least one solenoid for the generation of a pulsed electromagnetic field M(t) addressed towards a portion of human/animal tissue containing cartilage, characterised in that said electromagnetic field has a peak intensity between 0.5 and 2 milliTesla.

### BRIEF DESCRIPTION OF THE DRAWINGS

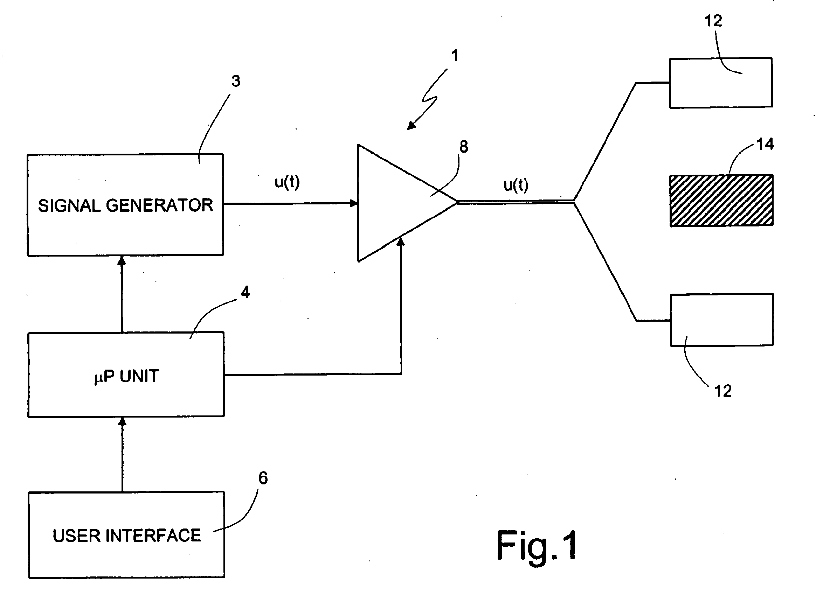

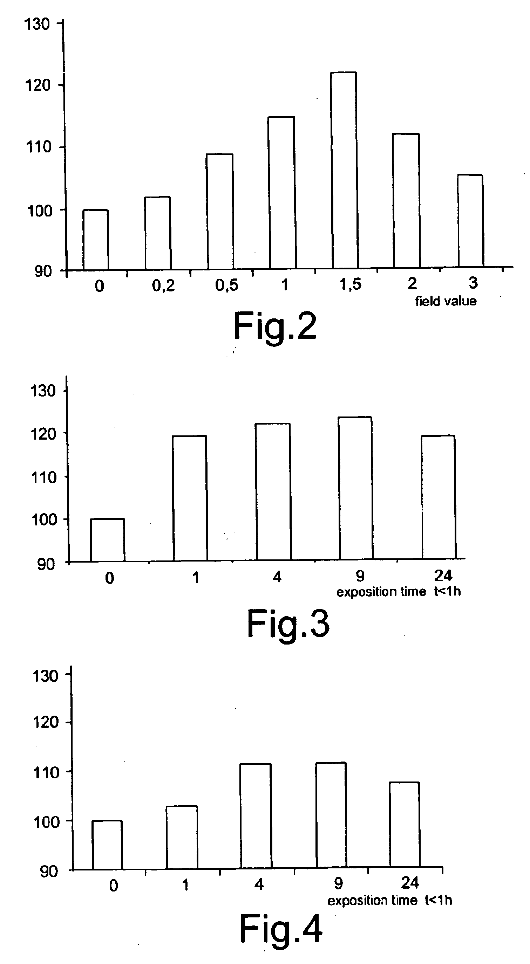

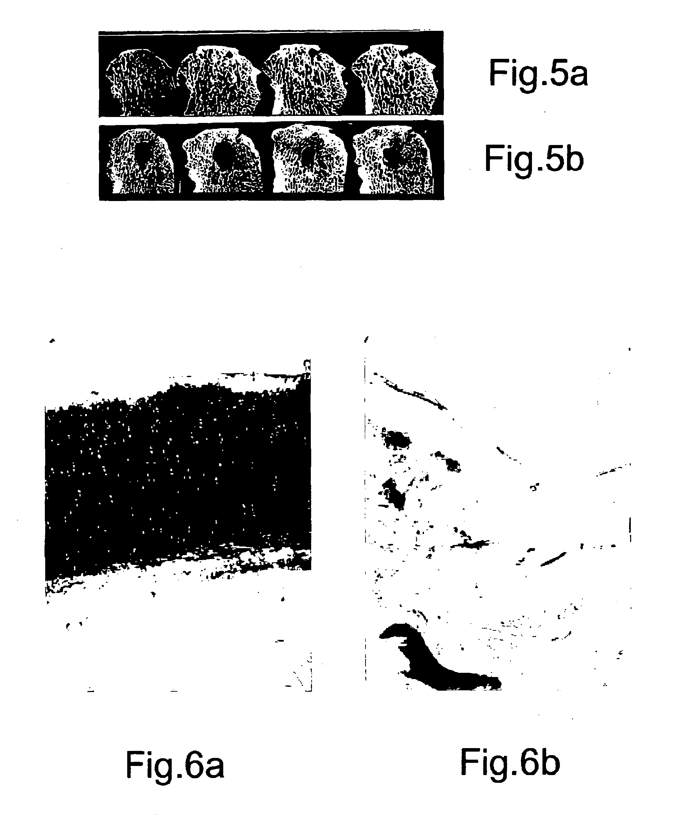

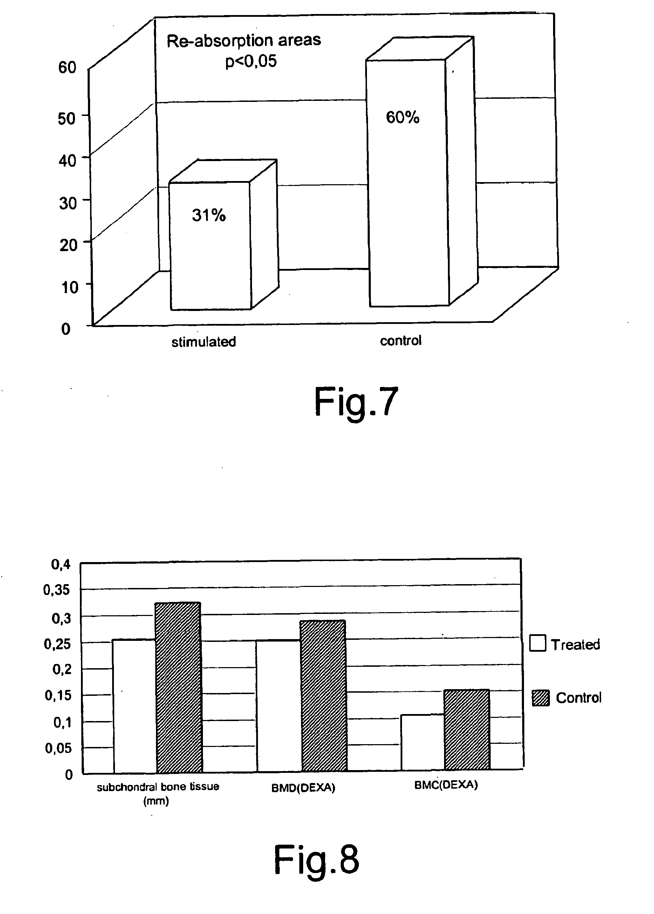

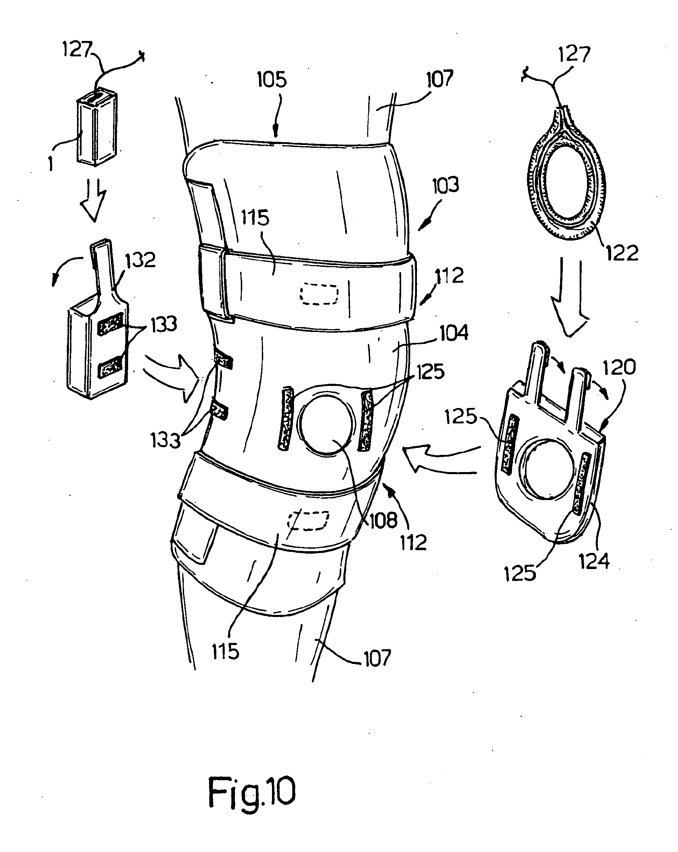

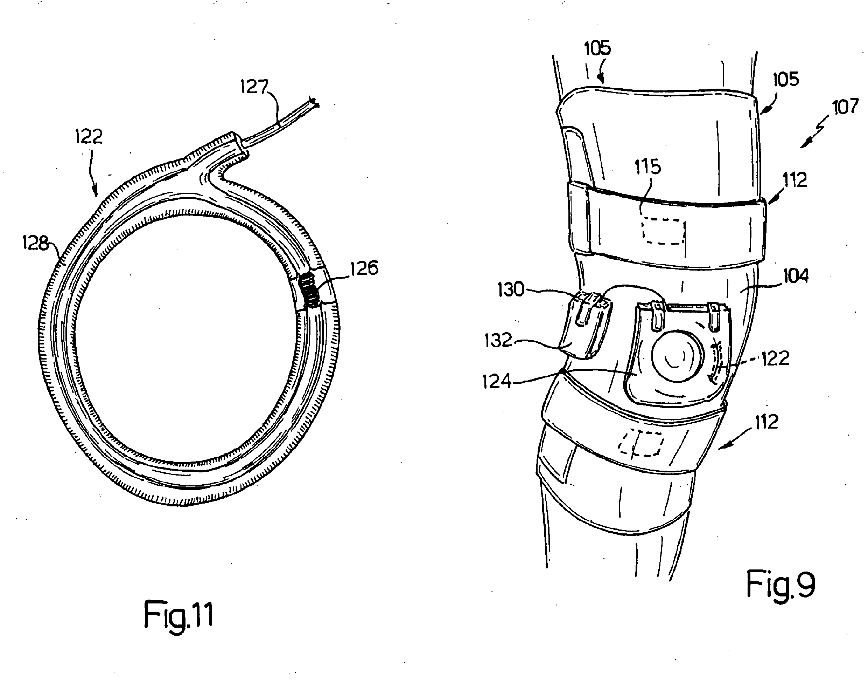

The invention will now be illustrated with particular reference to the accompanying drawings which represent a preferred non-limiting embodiment in which:
➢ a device for the regeneration and prevention of degeneration of the cartilaginous tissue and subchondral bone and the proliferation of chondrocytes by means of a pulsed electromagnetic field produced according to the precepts of the present invention;
➢ figures 2, 3 and 4 illustrate parameters that can be controlled figure 1 illustrates a simplified wiring diagram of by the device of the present invention;
➢ figures 5a, 5b, 6a and 6b illustrate results obtained with the device of the present invention;
➢ figures 7 and 8 illustrate statistical analyses performed on the data obtained with the device of the present invention;
➢ figures 9, 10 and 11 show support means to make the device of figure 1 portable by a person.

### BEST MODE FOR CARRYING OUT THE INVENTION

With reference to figure 1, 1 indicates, as a whole, a device for the regeneration and prevention of degeneration of the cartilaginous tissue and subchondral bone and the proliferation of chondrocytes by means of a pulsed electromagnetic field.

The device 1 comprises a signal generator device 3 (of known type) controlled by a microprocessor unit 4 and suitable for outputing a periodic type signal u(t).

The microprocessor unit 4 is connected to a user interface 6 (for example keyboard/video) for selection of the waveform (square wave, saw tooth, linear ramp, etc.) of the signal u(t) and adjustment of the frequency and duty-cycle of said signal u(t). The user interface 6 also permits generation of the signal u(t) for a time interval Tmax that can be selected as required.

The signal generator device 3 is connected at the output to the input of a variable gain power amplifier 8 which outputs a power signal U(t) transmitted to a pair of solenoids or to one single solenoid (of known type) 12, appropriately curved and modelled. In some embodiments the solenoid 12 can be one single solenoid.

During use, a portion of human/animal body containing a portion of cartilage/subchondral bone 14 to be treated is positioned between the pair of solenoids 12. A culture (not illustrated) of chondrocytes can also be positioned between the solenoids 12.

The power amplifier 8 is controlled by the microprocessor unit 4 so that via the user interface 6 it is possible to adjust the amplitude of the signal U(t) transmitted to the solenoids and therefore the intensity of the electromagnetic field M(t) acting on the cartilage 14.

Preferably but not exclusively each solenoid 12 consists of a support made of a sheet of flexible material on which a trace of conductive material (for example copper) is deposited, forming the coils of the solenoid. Alternatively, the solenoid could be made with a reduced number of coils (for example below 200) so that it can be modelled for good adhesion to the articular surface.

According to the present invention it is possible, via the user interface and the microprocessor unit 4, to set the device 1 so that it generates a pulsed electromagnetic field with peak intensity between 0.5 and 2 milliTesla.

The medical studies carried out by the applicant have highlighted that the identification of said limited power interval (0.5 and 2 milliTesla) permits generation of an electromagnetic field M(t) which results in effective regeneration and/or prevention of degeneration of the cartilage and subchondral bone.

In further detail, the electromagnetic field can have a power of between 1 and 2 milliTesla.

In particular the electromagnetic field can have a power of around 1.5 milliTesla, as highlighted in figure 2 which shows on the X axis the value of the electromagnetic field and on the Y axis the synthesis of proteoglycans which indicate an anabolic effect on the cartilage and in particular on the cartilaginous matrix; the value of 1.5 m-Tesla permits maximisation of regeneration and prevention of degeneration of the cartilage and subchondral bone.

As is known, a high synthesis of proteoglycans is an indicator of synthesis activity of the extracellular matrix of the articular cartilage.

The studies of the applicant have shown that correct adjustment of the field intensity is the main factor for obtaining a correct process of regeneration and prevention of degeneration of the cartilage and subchondral bone.

The studies of the applicant have also shown that, subordinately to the intensity, also the frequency of the signal applied constitutes a factor for control of the processes of regeneration and prevention of degeneration of the cartilage and subchondral bone.

Indeed, according to a further embodiment of the present invention it is possible, via the user interface and the microprocessor unit 4, to set the device 1 so that it generates a signal u(t) having variable frequency below 100 Hz.

The studies carried out by the applicant show that a signal with frequency above 100 Hz results in an inefficient process of regeneration and prevention of degeneration of the cartilage and subchondral bone.

Preferably, the frequency of the signal u(t) is set so as to present a frequency of between 2 Hz and 75Hz.

In further detail, the frequency can be between 37Hz and 75Hz, the frequency interval in which the greatest therapeutic effect is produced.

The interval 37-75 Hz permits maximisation of the processes of regeneration and prevention of degeneration of the cartilage and subchondral bone as highlighted in figure 3 which shows on the X axis the frequency value of the signal u(t) and on the Y axis the synthesis of the proteoglycans.

Lastly, the studies of the applicant have highlighted that, subordinately to the intensity and frequency, the duration of the treatment also constitutes a factor for control of the processes of regeneration and prevention of degeneration of the cartilage and subchondral bone.

In particular, via the user interface and the microprocessor unit 4, the device 1 is set so that it generates an electromagnetic field for a variable time interval, if possible less than 9 hours. Preferably the setting interval of the electromagnetic field is between 4 and 9 hours as highlighted in figure 4 which shows on the X axis the application time (expressed in hours) and on the Y axis the synthesis of proteoglycans.

### EXPERIMENTAL RESULTS

The method was tested in vitro on bovine cartilage which has a high affinity with human cartilage.

Explants of articular cartilage in the form of discs were performed from five different animals aged between 14 and 18 months.

In particular, four explants were performed on each donor animal taken from areas near the same joint thus obtaining twenty discs.

Each group of explants was divided at random into a first subgroup of explants with test function (therefore subject to the electromagnetic field) and a second group of explants with control function (therefore not subject to the electromagnetic field).

The explants underwent pre-treatment by placing them for 48 hours in a culture of DMEM/F12 to which 10% of FBS (Fetal Bovine Serum) and antibiotics (penicillin 100 units/ml, streptomycin 0.1 mg/ml) (Life Technologies Paisley, U.K.) were added.

Subsequently the explants were placed for an additional period of 48 hours in a medium without serum at 37°C in an atmosphere containing 5% of CO₂.

During the treatment each cartilage disc was placed between the solenoids 12 so that the plane of the solenoids was perpendicular to the discs and the direction of the electric field induced in the disc was perpendicular to the electromagnetic field.

The device 1 was used adjusting the intensity of the electromagnetic field, the frequency of the signal u(t) and the application time as illustrated above. The intensity of the electromagnetic field produced was measured with a Hall effect sensor of a gaussmeter.

In said regard, at the end of the period of equilibrium in culture illustrated above, the explants were exposed for: 1, 4, 9, 24 hours to a pulsed electromagnetic field obtained with the device 1.

Exposure to the pulsed electromagnetic field was performed with 10% FBS in the culture medium (0.5 ml/well). The evaluations were performed after 24 hours, independently of the exposure period.

The cultures not exposed to pulsed electromagnetic field (control cultures) were arranged in the same incubator as the one containing the cultures subject to electromagnetic field.

Synthesis of the proteoglycans was measured by incorporating a radioactive sulphate into the glycosaminoglycans (GAGs) which, as is known, are basic biochemical components of the proteoglycans.

The radioactive compound 5 µCi/ml of Na₂-³⁵SO₄ (2.2 mCi/ml) (produced by the company Amersham Pharmacia Biotech, Buckinghamshire, England) was added at time 0 both to the explants subject to treatment by pulsed electromagnetic field and to the control explants not subject to pulsed electromagnetic field, thus performing radio-marking.

After the radio-marking, the explants were washed and digested in a buffer containing 20 mM of phosphate (pH 6.8) and 4 mg/ml of papain (produced by the company Sigma-Aldrich S.r.l. Milan, Italy) and kept at 60°C for 12 hours.

The content of the proteoglycans marked with compounds of radioactive sulphur ³⁵S belonging to new synthesis proteoglycans PGs (³⁵S-PGs) was measured following precipitation of the radioactive sulphur compound ³⁵S-PGs by means of cetylpyridinium chloride (said compound is available from Sigma-Aldrich S.r.l. Milan, Italy) and filtering on fibreglass (Whatman GF/C).

The filters were then dried and the radioactive sulphur compounds were quantified by scintillator count. The quantity of proteoglycans synthesised as a result of the cellular activity or activity of the chondrocytes was thus identified.

On the basis of the results of the experiments an exposure time of between 1 hour and 9 hours was identified. In further detail, the maximum therapeutic effect is obtained with an exposure field of between 4 and 6 hours.

Subsequently synthesis of the proteoglycans was measured in the explants of cartilage using pulsed magnetic fields having different peak values of between 0.2mT and 3mT.

This permitted selection of the interval between 0.5 and 2 mTesla which defines a first therapeutic treatment window. The results of the tests also permitted definition of the sub-interval between 1 and 2 mT (second treatment window) and the peak value of 1.5 mT which maximises the effects of the treatment.

Lastly, following selection of the best exposure time and preferred electromagnetic field value, synthesis of the proteoglycans was measured with different frequencies (0, 1, 2, 37, 75, 110, 150, 200 Hz). This enabled us to ascertain that for frequencies above 100 Hz no appreciable therapeutic effects are obtained.

The sub-interval between 2 and 75 Hz and the sub-interval between 37 and 75 Hz in which the therapeutic effect is maximised were then selected.

On the basis of the results obtained by means of a first set of experiments, the explants were exposed for 9 hours to a pulsed electromagnetic field, the amplitude of which was around 1.5 milliTesla.

For said pulsed field value an unexpected synthesis of proteoglycans was found (approx. 50% more in the implants subject to pulsed electromagnetic field compared to the findings for the implants not subject to electromagnetic field).

Once the most effective window for each parameter of the pulsed electromagnetic field had been identified, the investigations were extended.

The studies performed by the applicant on sheep which underwent osteochondral transplant of the knee also showed that the action of the device 1 according to the present invention determines a rapid recovery of the subchondral bone tissue and prevents bone re-absorption phenomena, creating optimal conditions for viability and survival of the overlying articular cartilage.

Good integration of the transplanted bone tissue prevents the formation of small bone cysts thus guaranteeing stability of the bone graft in the long term. In this regard it should be noted that, in the case of osteocartilaginous transplants, early fixing of the subchondral bone is the necessary condition for viability and preservation of the cartilage transplanted and success of the operation.

Figures 5a, 5b illustrate radiographic images of an osteocartilaginous graft.

In particular, figures 5a refer to an osteocartilaginous graft stimulated with device 1: in said figures the optimal integration of the transplant throughout the thickness as shown by the different sections can be observed.

Figures 5b refer to an osteocartilaginous graft not stimulated with device 1: in said figures areas of re-absorption in the different sections can be observed.

In particular in the microradiographic image of figure 5a complete integration of the subchondral bone can be noted.

The percentage of bone re-absorption areas (dark) in the transplanted cylinders of the stimulated animals is 31%, against 60% bone re-absorption areas in the transplanted cylinders of the control animals.

The histogram of figure 7 illustrates the percentage of bone re-absorption areas present in the transplanted cylinders of the stimulated and control animals.

This figure shows that the pulsed electromagnetic fields are able to promote early fixing of the graft, guaranteeing optimal integration of the transplanted bone tissue, preventing the formation of small bone cysts, hence ensuring stability of the bone graft and therefore success of the operation.

The histological images 6a, 6b furthermore illustrate a section of the transplanted cartilage (figure 6a) treated with the device of figure 1 in which the viability of the transplanted cartilage, which has an adequate thickness and intense colouring of the cartilaginous matrix, is evident.

In particular figure 6a highlights the presence of hyaline tissue, while figure 6b (non-treated cartilage) highlights the presence of fibrous, fibrocartilaginous tissue.

In the transplants treated with the device 1 the formation of fibrous tissue is clearly inferior with respect to the non-treated controls: 15% as against 32%.

Lastly, the applicant has demonstrated that treatment with the device 1 can effectively prevent cartilaginous degeneration in experimental animals (Dunkin Hartley), maintaining functionality. Animals with initial osteoarthrosis, aged 15 months, were treated for 6 months. Not only did treatment with the device 1 prevent degeneration of the cartilage, it also prevented osteosclerosis of the subchondral bone, indicating that the cartilage had maintained its mechanical characteristics. Indeed, when the cartilage loses the ability to absorb stress due to the load, the stress is transmitted directly to the subchondral bone tissue which reacts by increasing its density and thickness. The table shows that the histological evaluation (Mankin score) of the cartilage treated with the device 1 is clearly inferior (therefore better) than in the control animals.

| | Control animals | Animals treated with electromagnetic fields |
|---|---|---|
| Mankin score | 13.8 ± 1.1 | 4.6 ± 1.5*** |

The histomorphometric and bone density measurements by means of Dual Energy X-ray Absorptiometry (DEXA) highlight a lesser density and sclerosis of the subchondral bone tissue in the animals treated with the device 1 (figure 8).

Lastly, experiments carried out by the applicant have highlighted that the electromagnetic field generated by the device 1 according to the above procedures is effective in stimulating the proliferation of chondrocytes cultivated in vitro.

Said chondrocytes can be used in different techniques, for example they can be used to perform Autologous Chondrocyte Implantation (ACI), a method introduced in the eighties by Petterson to promote healing of the cartilage.

Said method provides for an initial collection of autologous chondrocytes by means of arthroscopy from patients affected by chondral lesions. The chondrocytes are then isolated by digestion of the cartilaginous matrix and cultivated in vitro, after dedifferentiation towards the chondroblastic phenotype. In the ACI technique, the cells thus obtained are then transplanted, in the form of suspension, into the patient's joint below a periostal flap sutured to the chondral cartilage during the operation.

Alternatively, the chondrocytes can be used in the MACI (Matrix-Induced Autologous Chondrocyte Implantation) technique which involves initial arthroscopy and in vitro cultivation of autologous chondrocytes: the chondroblasts thus obtained are scattered three weeks after collection on a type I and III pig collagen scaffold. This "membrane" can be implanted on the chondral lesion of the patient and affixed via the use of fibrin glue.

The applicant has been able to demonstrate that on the one hand treatment with the device 1 (and with the parameters highlighted above) stimulates the proliferation of these cells which are transplanted. Stimulation of proliferation represents a fundamental element for colonisation of the cartilaginous lesion site to be treated.

Lastly it is important to remember the role of the stem cells in the healing process of a cartilaginous lesion, as demonstrated by the techniques that involve making small perforations in the subchondral bone at the base of cartilaginous lesions. The aim is to promote the migration of totipotent cells, from the bone marrow to the surface of the subchondral bone, so that they can provide the necessary biological support for healing.

The applicant has carried out studies on stem cells obtained via the process briefly illustrated above, in order to highlight that the device is able to stimulate the proliferation, migration and ability thereof to colonise a substrate used in the treatment of cartilaginous lesions. According to a preferred and independent aspect of the present invention, the device 1 is coupled with support means to make the device 1 portable by a person.

These supporting means comprise (figure 9, 10 and 11) a supporting body 103 defined by at least one contoured wall 104 defining a cavity 105 for housing a portion of the human body.

In the non-limiting example shown, wall 104 is shaped to define a kneepad, which defines the elongated cavity 105 for housing a leg portion 107 of a patient (not shown in full) close to the knee 108. Cavity 105, however, may obviously house any portion of the human body, e.g. an arm, shoulder, etc.. Wall 104 is preferably made of flexible synthetic material to adapt to the contour of the human body, and is obviously also made of anti-allergic, nontoxic material, such as neoprene.

Supporting means also comprises an elastic connecting device 112 fitted to supporting body 103 to secure contoured wall 104 firmly to the portion of the human body.In the example shown, the elastic connecting device comprises two elastic straps 115, each having a portion fixed (e.g. stitched) to wall 104, and each having a fastening device, e.g. of VELCRO™, at the ends. Fastening devices other than those shown, however, may obviously be used.

Supporting means also comprises a seat 120 for housing a the solenoid 12 of device 1 located adjacent to contoured wall 104 and therefore close to the portion of the human body.

In the example shown, seat 120 is defined by a square pouch structure 124 made of fabric and connectable to an outer surface of contoured wall 4 by two reversible connecting devices 125, e.g. of VELCRO™, so that seat 120 is secured firmly in a predetermined position to contoured wall 104 when connecting devices 125 are connected firmly.

Solenoid 12 is conveniently made using a coiler (not shown), which forms a coil 126 (Figure 11) comprising roughly 200 turns of copper wire with an average turn of 40 cm. Coil 126 is then wound with cotton tape to keep its shape. The two ends of coil 126 are connected to a bipolar power cable 127 of solenoid 122. Coil 126 is then covered with heat-sealed multilayer plastic material 128 comprising high-density sponge inside and a PVC sheet outside. Solenoid 122 is wound in air.

Solenoid 12 is conveniently powered by the device 1, which may also be housed in a pouch 132 fixed to the outer surface of wall 104 by a releasable connecting device 133, e.g. of VELCRO™.

In a variation not shown, more than one seat may be formed to house further solenoids. For example, two separate seats may be formed for two solenoids located, in use, on opposite sides, one medial and one lateral, of the joint for treatment, which is an advantageous arrangement for treating the knee joint. A single solenoid is mainly indicated for treatment of the kneecap, and two solenoids for treatment of larger areas of the joint or for patients with larger than average joints. Two solenoids therefore ensure a uniform induced signal over the whole joint, even in patients with more extensive lesions.

## Claims

1. Device for the regeneration and prevention of degeneration of the cartilage and subchondral bone and proliferation of chondrocytes by means of electromagnetic waves comprising:
- means (3) for generating a periodic signal u(t);
- power amplifying means (8) suitable for applying said signal u(t) to at least one solenoid (12) for the generation of a pulsed electromagnetic field M(t) addressed towards a portion of human/animal tissue containing cartilage,
**characterised in that** it comprises setting means (6,4) configured to generate an electromagnetic field having peak intensity of around 1.5 milliTesla.

2. Device as claimed in claim 1, wherein said setting means (6,4) contribute to generating a periodic signal u(t) having frequency below 100 Hz.

3. Device as claimed in claim 1, wherein said setting means (6,4) contribute to generating a periodic signal u(t) having frequency of between 2 and 75 Hz.

4. Device as claimed in claim 1, wherein said setting means (6,4) contribute to generating a periodic signal u(t) having frequency of between 37Hz and 75 Hz.

5. Device as claimed in claim 1, wherein said setting means (6,4) contribute to generating an electromagnetic field for a time interval of under 9 hours.

6. Device as claimed in claim 1, wherein said setting means (6,4) contribute to generating an electromagnetic field for a time interval of between 4 and 9 hours.

7. Device as claimed in claim 1, wherein support means are provided to make the device (1) portable by a person.

## Patentansprüche

1. Vorrichtung zur Regeneration und Verhinderung von Degeneration des Knorpels und des subchrondralen Knochens und der Proliferation von Chondrozyten mittels elektromagnetischer Wellen, mit:
- einer Einrichtung (3) zum Erzeugen eines periodischen Signals u(t);
- einer Energieverstärkungsvorrichtung (8), die geeignet ist zum Zuführen des Signals u(t) zu mindestens einem Elektromagneten (12) zwecks Erzeugens eines gepulsten elektromagnetischen Felds M(t), das auf einen Knorpel enthaltenden Teil menschlichen/tierischen Gewebes gerichtet wird,
**dadurch gekennzeichnet, dass** die Vorrichtung Einstelleinrichtungen (6,4) aufweist, die zum Erzeugen eines elektromagnetischen Felds mit einer Peak-Intensität von ungefähr 1,5 Millitesla konfiguriert sind.

2. Vorrichtung nach Anspruch 1, bei der die Einstelleinrichtungen (6,4) zum Erzeugen eines periodischen Signals u(t) mit einer Frequenz unterhalb 100 Hz beitragen.

3. Vorrichtung nach Anspruch 1, bei der die Einstelleinrichtungen (6,4) zum Erzeugen eines periodischen Signals u(t) mit einer Frequenz zwischen 2 und 75 Hz beitragen.

4. Vorrichtung nach Anspruch 1, bei der die Einstelleinrichtungen (6,4) zum Erzeugen eines periodischen Signals u(t) mit einer Frequenz zwischen 37 Hz und 75 Hz beitragen.

5. Vorrichtung nach Anspruch 1, bei der die Einstelleinrichtungen (6,4) zum Erzeugen eines elektromagnetischen Felds während eines Zeitintervalls von weniger als 9 Stunden beitragen.

6. Vorrichtung nach Anspruch 1, bei der die Einstelleinrichtungen (6,4) zum Erzeugen eines elektromagnetischen Felds während eines Zeitintervalls zwischen 4 und 9 Stunden beitragen.

7. Vorrichtung nach Anspruch 1, bei der Haltemittel vorgesehen sind, um die Vorrichtung (1) von einer Person tragbar zu machen.

## Revendications

1. Dispositif pour la régénération et la prévention contre la dégénération du cartilage et de l'os sous-chondral et la prolifération de chondrocytes par l'intermédiaire d'ondes électromagnétiques comprenant :
- des moyens (3) pour générer un signal périodique u(t) ;
- des moyens d'amplification de puissance (8) adaptés pour appliquer ledit signal u(t) à au moins un solénoïde (12) pour la génération d'un champ électromagnétique pulsé M (t) dirigé vers une partie de tissu humain/animal contenant du cartilage,
**caractérisé en ce que** il comprend des moyens de paramétrage (6, 4) configurés pour générer un champ électromagnétique ayant une intensité de crête d'environ 1,5 milliTesla.

2. Dispositif selon la revendication 1, dans lequel lesdits moyens de paramétrage (6, 4) contribuent à générer un signal périodique u(t) ayant une fréquence située au-dessous de 100 Hz.

3. Dispositif selon la revendication 1, dans lequel lesdits moyens de paramétrage (6, 4) contribuent à générer un signal périodique u(t) ayant une fréquence comprise entre 2 et 75 Hz.

4. Dispositif selon la revendication 1, dans lequel lesdits moyens de paramétrage (6, 4) contribuent à générer un signal périodique u(t) ayant une fréquence comprise entre 37 et 75 Hz.

5. Dispositif selon la revendication 1, dans lequel lesdits moyens de paramétrage (6, 4) contribuent à générer un champ électromagnétique pour un intervalle de temps inférieur à 9 heures.

6. Dispositif selon la revendication 1, dans lequel lesdits moyens de paramétrage (6, 4) contribuent à générer un champ électromagnétique pour un intervalle de temps compris entre 4 et 9 heures.

7. Dispositif selon la revendication 1, dans lequel des moyens de support sont fournis pour rendre le dispositif (1) portable par une personne.
